# EUROPEAN PATENT APPLICATION

(11) **EP 1 764 059 A1**
(43) Date of publication of application: **21.03.2007**
(21) Application number: 05255763.4
(22) Date of filing: 16.09.2005
(51) Int. Cl.: A61B 19/00

(54) **Navigable instrument**

(71) Applicant: Depuy International Limited, Beeston, Leeds LS11 8DT (GB); DePuy Orthopädie GmbH, 85551 Heimstetten (DE)
(72) Inventor: Warnock, Alex, Feldkirchen 85622 (DE); Ashby, Alan, York YO30 6LN (GB)
(74) Representative: Alton, Andrew

(57) **Abstract**

A navigable instrument for use with a tracking part of a computer aided surgery system is described. The instrument includes a body to which a line of sight trackable marker can be attached. A working part of the instrument has a working point and the working part is rotatably attached to the body and configured such that the position of the working point does not move relative to the body as the working part is rotated relative to the body.

## Description

The present invention relates to a surgical instrument, and in particular to a navigable surgical instrument which is easier to use with a tracking part of a computer aided surgery system.

Computer aided surgery (CAS) systems often include a tracking system by which the real time positions of various entities used with the CAS system, e.g. instruments, tools, implants, body parts, etc., can be determined and displayed to the user in conjunction with an image or images of the patient, or a model of the patient, so as to provide image guided surgery. Various tracking technologies can be used including wired and wireless tracking technologies. Many wireless tracking technologies require a line of sight between markers, also referred to as fiducial markers, attached to the entity being tracked and a detector.

For example, ultrasound based tracking technologies can require a line of sight between ultrasound emitting markers and ultrasound detectors so that the position of the markers can be determined from time of flight measurements of the ultrasound transmitted by the markers. Similarly light based tracking systems, such as those using infra red radiation, can require a line of sight between reflective markers and a pair of off set cameras which capture stereoscopic infra red images of the markers from which the position of the markers can be determined as is known in the art. Other light based tracking technologies can be based on active markers which emit light which is captured by detectors, such as cameras.

Irrespective of the underlying transmission medium, for such systems which rely on radiation (whether electromagnetic or acoustic) being able to pass uninterrupted between the marker and the detectors, reliable tracking requires uninterrupted paths between the markers and detectors. Otherwise the signal is lost and so the current position of the entity may be lost and/or when the interruption is removed, the tracking system may not be able to determine where the entity has moved to. Various events can cause the signal to be interrupted by blocking the line of sight between the markers and detectors, such as parts of the body of the patient, obscuring the markers, parts of the body of the operating theatre staff, parts of operating theatre equipment, other entities being tracked and also the attitude, or orientation, of the entity being tracked relative to the detectors.

Some entities have a preferred direction in which they need to be used by the user, for example owing to the anatomy of the patient or spatial constraints owing to the surgical procedure, e.g. in minimally invasive surgery. However, there can be a conflict between being able to use the entity in the preferred direction while still maintaining a line of sight between the markers on the entity and the detectors. One option is to move the detectors during the surgical procedure, but that will incur delays and may result in other tracked entities no longer being in line of sight communication with the detectors. Another option is to remove the markers from the entity and reattach the markers at a different position. However, this also incurs delays and also may result in the entity requiring recalibration to reestablish the positional relationship between the markers and the part of the instrument being navigated. It is important to avoid or reduce delays during surgical procedures, for example to help reduce the chance of infection.

Further, there may be some circumstances in which the user wants to use the instrument in a certain direction, but geometrical constraints imposed by the environment prevents the user using the tracked instrument in that direction. For example, a body part adjacent the surgical site may get in the way of the marker and so prevent the instrument from being used in the correct direction.

Therefore, it would be advantageous to be able to provide an instrument which can easily and quickly be used in its preferred direction while reliably being tracked.

According to a first aspect of the present invention, there is provided a navigable instrument for use with a tracking part of a computer aided surgery system, the instrument comprising a body to which a marker can be attached and a working part of the instrument having a working point, the working part being rotatably attached to the body and configured such that the position of the working point does not move relative to the body as the working part is rotated relative to the body.

There is a fixed positional relationship between the working point, which is to be navigated, and the part of the instrument to which a trackable marker can be attached, while the angular position of the working part can be changed, it is possible quickly and easily to configure the instrument for use in a preferred direction while taking into account any limitations imposed by the geometry of the surgical site or environment. For example, the marker can be repositioned to prevent the marker from clashing with a body part while being used in the preferred direction.

The working point does not need to correspond physically to a point, but rather can be a point which can be used in practice to characterises the location of the working part in space. For example, if the instrument were a scalpel, then the working part would be the blade and the working point could be any point on the blade which can be used to characterise the position of the blade in use, such as the tip of the blade or a particular position on the edge of the blade.

The marker can be a line of sight marker. In this way, the instrument can be used in a preferred direction while still providing line of sight communication between the marker and the tracking system.

Preferably, the working part is configured such that the working point lies on the axis of rotation of the working part.

The instrument can be any suitable navigable instrument having a preferred direction of use. For example, the instrument can be a pointer. The working part can be a probe of the pointer and the tip of the probe can be the working point of the instrument. The tip of the instrument can be located on an axis of rotation of the working part.

The probe can include a main portion and an end portion which is curved or bent relative to the main portion. At least some or the whole of the main portion of the probe can be offset from an axis of rotation of the working part. The tip of the probe can be located on the axis of rotation.

The body can provide a handle or a part of the handle of the instrument.

The instrument can include a line of sight trackable marker attached to the body or a part of the body. The marker can be permanently attached or releasably attached. The body can include a formation or formations to which the marker can be releasably attached. The marker can comprise a plurality of marker elements and can be in the form of a marker array or star array, such as, for example a T array or a Y array.

The working part can be indexed so that the working part can be rotated between separate discrete angular positions. The discrete angular positions can be equi-angularly spaced. The indexing mechanism can prevent or help prevent free rotation of the working part relative to the body. The indexing mechanism can define any number of separate positions. Preferably, the indexing mechanism defines a number of separate positions in the range from four to twenty, more preferably in the range from eight to sixteen and most preferably twelve positions.

The indexing mechanism can be provided by a resiliently biassed member and a plurality of formations with which the resiliently biassed member can individually engage. Preferably the formations are female formations, such as indents or recesses, and the biassed member is a male formation, such as a nub, curved peg or tip. The indexing mechanism can be provided at an interface between the working part and the body of the instrument. The formations can be provided on an inner or reverse side of a disc portion of the working part. The disk portion can provide a closure to an open end of an at least partially hollow body part.

The working part can include a grip. The grip can be manipulated by a user to rotate the working part relative to the body. The grip can include a plurality of formations around the periphery of a part of the working part. The grip can be provided by knurls.

A portion of the working part can extend into the body. The portion extending into the body can act to secure the working part to the body. The working part can be secured to the body at least partially by an O-ring and opposed grooves. The o-ring can engage a circular groove on an inner surface of the body and an opposed groove on an outer surface of the portion of the working part.

The instrument can further include a shaft extending from the working part into the body. The shaft can extend along a the rotational axis of the working part so as to support the working part.

An embodiment of the invention will now be described, by way of example only, and with reference to the accompanying drawings, in which:
Figure 1 shows a perspective view of an instrument according to the invention;
Figure 2 shows a perspective partially exploded view of the instrument shown in Figure 1; and
Figure 3 shows a magnified partial cross sectional view of part of a further embodiment of the instrument of the invention.

Similar items in different Figures share common reference numerals unless indicated otherwise.

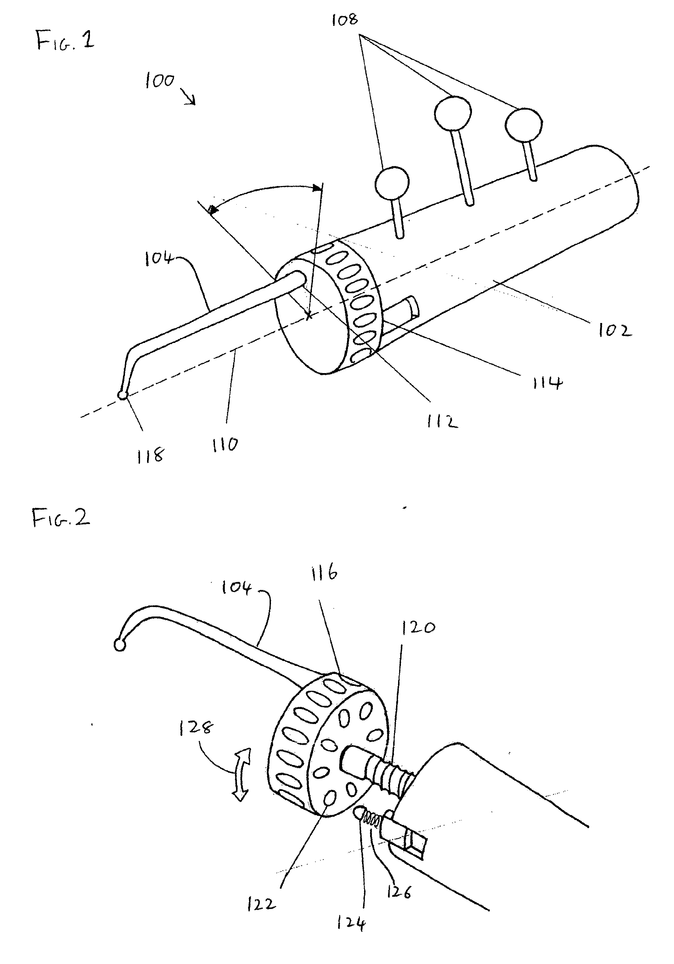

Figure 1 shows a perspective view of an instrument 100 according to the invention. Instrument 100 is in the form of a pointer. Instrument 100 includes a right circular cylindrical hollow body 102 which provides a handle of the pointer. Body 102 is closed at a proximal end and open at a distal end. A marker 108 is permanently and statically attached to the body in the form of three reflective spheres mounted on posts of different heights. The posts are arranged in a straight line parallel to a longitudinal axis 110 of the body. The longitudinal axes of the posts are also directed toward axis 110.

Instrument 100 also includes a working part in the form of a probe 104 which is rotatably mounted to the body 102 so as to rotate about axis 110. Probe 104 includes a stem 112 which is mounted on a generally circular disk 114 which has a plurality of raised and recessed formations or knurling 116 around its periphery to provide a grip. Other embodiments may use different types of formations to provide a grip to facilitate manual rotation of the working part. Probe 104 terminates in a tip 118. Tip 118 is the working point of the instrument, being the part of the instrument which characterises the use of the instrument and which is navigated in use. The stem 112 of the probe is off set from the axis of the instrument and the working part is configured such that the tip 118 is positioned on the rotational axis 110.

As illustrated in Fig.2, which is an exploded perspective view of the instrument 100, a shaft 120 extends from a rear side of disk 114 and extends inside the hollow body. A mounting is provided in the end wall of the body 102 and which rotatably receives the free end of shaft 120 so as to provide support for the shaft and also acts to retain the working part against the open end of the body 102. As also illustrated in Figure 1, the instrument is provided with an indexing mechanism. A plurality of equi-angulalrly spaced recesses or indents 122 are provided around the inner face of disk 114. In the illustrated embodiment, eight indents 122 are provided, with an angle of approximately 45 between each neighbouring pair. However, in other embodiments different numbers of indents can be provided to provide a coarser (eg four indents) or finer scale (e.g. twelve indents) of indexing. A plunger having a domed tip 124 mounted on a spring 126 is provided as part of the wall of body 102. The plunger is positioned so that the domed tip can sequentially engage each of the indents 122 as the working part is rotated relative to the body 102 and the spring 126 resiliently biasses the tip 124 to engage with the indents.

As illustrated by arrow 128, the working part of the instrument can be rotated in a clockwise or counterclockwise direction relative to the body. In use, the surgeon may use the pointer to identify the position of an anatomical feature of the patient, for example as part of a registration process, to the tracking system. At least the tip part of the probe 104 of the pointer is curved so that the pointer has a preferred direction in which it can be used when held, for example to gain access to a recessed anatomical feature, or so as to improve the ergonomic performance of the pointer. However, as the probe can be rotated relative to the body to which the marker 108 is attached, the surgeon can simply rotate the probe by manipulating knurls 116 so that, with the tip of the probe pointing in the preferred direction, the markers will still be in line of sight contact with the cameras of the tracking system.

As the working part is configured so that the tip 118 of the probe is positioned on the axis of rotation 110 of the working part, there is a fixed positional relationship between the tip 118 and the marker 108. Therefore, the position of the tip of the instrument can be reliably determined by the tracking system without needing to re-calibrate the instrument, and irrespective of the angular relationship between the marker 108 and the working part. Hence, the instrument can be reliably navigated in its preferred orientation in use. The indexing allows a specific angle to be selected, if required, and also helps to prevent the working part of the instrument from rotating freely in use.

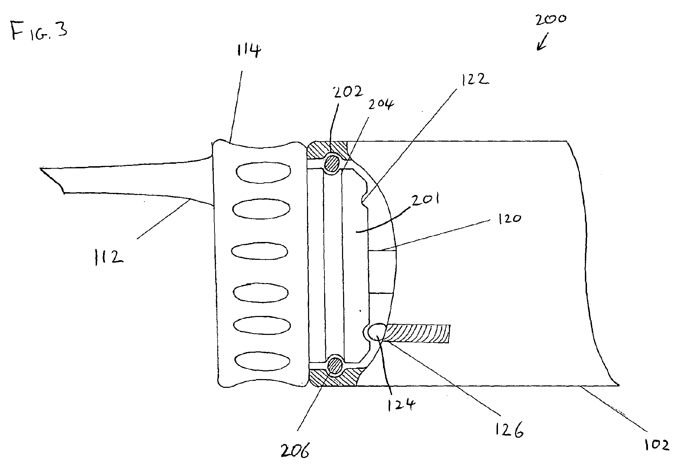

Figure 3 shows a magnified partial cross section of a further embodiment of an instrument 200 according to the invention. The instrument 200 is also a pointer and is in many respects similar to the instrument 100 shown in Figures 1 and 2 and so those parts will not be described in detail. However, the instrument 200 has a different mechanism coupling the working part to the body.

As shown in Figure 3, the reverse face of disk 114 includes a circular portion 201 extending away from the disk and into the free end of the hollow body 102. A first groove 202 extends around an inner surface of the body toward its free end. A second groove 204 extends around the portion 201 of the disk is opposed to the first groove. An O-ring 206 is provided in the gap defined between the first and second grooves. Hence, the O-ring acts to both seal the inner volume of the body 102 and also secures the working part to the body while allowing relative rotation between them.

It will be appreciated that the present invention is not limited to the specific instrument illustrated and can be used with any suitable instrument having a preferred direction and wherein the working part can be arranged so that the working point of the instrument (the tip of the pointer in the described embodiment) can be positioned to have a fixed position relative to the part of the body to which the marker is attached while the working part itself can have different angular positions. For example, the invention could also be applied to a scalpel, in which the scalpel blade is the working part and the tip of the blade, or a particular point on the edge of the blade, is the working point which can be used to characterise the position of the blade.

Further, the invention is not limited to instruments having a marker permanently attached thereto. The invention can also be used with instruments to which a marker (such as an array or star array) can be releasably attached, for example using a clip, a clamp or some other releasable fastening, provided that there is a fixed positional relationship between the working point of the instrument and the marker when attached.

## Claims

1. A navigable instrument for use with a tracking part of a computer aided surgery system, the instrument comprising:
a body to which a trackable marker can be attached; and
a working part of the instrument having a working point, the working part being rotatably attached to the body and configured such that the position of the working point does not move relative to the body as the working part is rotated relative to the body.

2. The instrument as claimed in claim 1, wherein the instrument is a pointer and the working part is a probe of the pointer and the body provides a handle of the pointer.

3. The instrument as claimed in claim 2, wherein a portion of the probe is offset from an axis of rotation of the working part relative to the body about which the working part can rotate and the tip of the probe is located on the axis of rotation.

4. The instrument as claimed in any preceding claim, wherein the instrument includes a line of sight trackable marker attached to the body.

5. The instrument as claimed in claim 4, wherein the line of sight trackable marker is permanently attached to the body.

6. The instrument as claimed in any preceding claim, wherein the working part is indexed so that the working part can be rotated between separate discrete angular positions.

7. The instrument as claimed in claim 6, wherein the working part is equi-angularly indexed.

8. The instrument as claimed in claim 6 or 7, wherein the indexing is provided by a resiliently biassed member and a plurality of formations with which the resiliently biassed member can individually engage.

9. The instrument as claimed in any preceding claim, wherein the working part includes a grip which can be manipulated by a user to rotate the working part relative to the body.

10. The instrument as claimed in claim 9, wherein a portion of the working part extends into the body and acts to secure the working part to the body.

11. The instrument as claimed in claim 10, wherein the working part is secured to the body at least partially by the action of an o-ring engaging a circular groove on an inner surface of the body and an opposed groove on an outer surface of the portion of the working part.

12. The instrument as claimed in any preceding claim, and further including a shaft extending from the working part into the body along a rotational axis of the working part about which the working part can rotate so as to support the working part.
